(19) Europäisches Patentamt
European Patent Office
Office européen des brevets

(11) **EP 1 745 770 A1**

(12) **DEMANDE DE BREVET EUROPEEN**


(43) Date de publication:
**24.01.2007 Bulletin 2007/04**

(51) Int Cl.:
***A61K 8/04*** (2006.01) ***A61K 8/06*** (2006.01)
***A61K 8/72*** (2006.01) ***A61K 8/81*** (2006.01)

(21) Numéro de dépôt: **06291033.6**

(22) Date de dépôt: **23.06.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **13.07.2005 US 698791**



(71) Demandeur: **L'Oreal**
**75008 Paris (FR)**

(72) Inventeurs:
- **Blin, Xavier**
  **75015 Paris (FR)**
- **Lu, Shaoxiang**
  **Plainsboro, NJ 08536 (US)**
- **Bui, Hy Si**
  **St Piscataway, NJ 08854 (US)**

(74) Mandataire: **Chantraine, Sylvie Hélène**
**L'ORÉAL D.I.P.I**
**25-29 Quai Aulagnier**
**92665 Asnières-sur-Seine Cedex (FR)**


(54) **Produit cosmétique bicouche, ses utilisations et kit de maquillage contenant ce produit**


(57) L'invention se rapporte à un produit cosmétique comprenant une première et une seconde compositions, la première composition contenant au moins une résine choisie parmi la colophane et ses dérivés, les résines hydrocarbonées et leurs mélanges, ladite résine présentant une masse moléculaire moyenne en nombre inférieure ou égale à 10 000 g/mol, et la seconde composition distincte de la première comprenant au moins un corps gras non volatile.

L'invention se rapporte également à un procédé de maquillage ainsi qu'à un kit de maquillage contenant ledit produit. Ce dernier est en particulier un rouge à lèvres, un mascara, un fond de teint ou un vernis à ongles.


EP 1 745 770 A1

## Description

La présente invention se rapporte à un produit cosmétique comprenant au moins deux compositions qui peuvent être appliquées successivement sur la peau aussi bien du visage que du corps, sur les paupières inférieures et supérieures, sur les lèvres et sur les phanères comme les ongles, les sourcils, les cils ou les cheveux. La présente invention concerne également un procédé de maquillage du visage et du corps mettant en oeuvre ces deux compositions.

Chaque composition peut être un fond de teint, un fard à joues ou à paupières, un produit anti-cerne, un blush, une poudre libre ou compactée, un rouge à lèvres, un baume à lèvres, un brillant à lèvres, un crayon à lèvres ou à yeux, un mascara, un eye-liner, un vernis à ongles ou encore un produit de maquillage du corps ou de coloration de la peau.

La présente invention a pour but de proposer une nouvelle voie de formulation d'un produit cosmétique, en particulier un produit de maquillage, du type comportant deux compositions à appliquer successivement l'une après l'autre. Ce produit cosmétique peut avantageusement présenter de bonnes propriétés de brillance et de tenue.

Ces propriétés de tenue, de non transfert et de non migration, alliées à l'aspect brillant et non gras, en font un produit particulièrement approprié pour réaliser des produits de maquillage des lèvres tels que rouges à lèvres, brillants à lèvres, des yeux tels que mascara, eye-liners, fards à paupières, des ongles ou des cheveux.

Un objet de la présente invention est donc un produit cosmétique destiné à être appliqué sur les matières kératiniques, comprenant une première et une seconde compositions, la première composition comprenant au moins une résine choisie parmi les colophanes, les dérivés de colophanes, les résines hydrocarbonées et leurs mélanges, ladite résine présentant une masse moléculaire moyenne en nombre inférieure ou égale à 10 000 g/mol, et la seconde composition distincte de la première comprenant au moins une huile de haute viscosité.

Le produit de l'invention est en particulier un produit de maquillage de la peau, des ongles ou des cheveux.

Par produit de maquillage, on entend un produit contenant un agent colorant permettant le dépôt d'une couleur sur une matière kératinique (la peau ou les phanères) de personne humaine par l'application sur la matière kératinique de produits tels que des rouges à lèvres, des fards, des eye-liners, des fonds de teint ou des autobronzants, des produits de maquillage semi permanent (tatouage).

Le produit selon l'invention comprend au moins deux compositions cosmétiquement acceptables conditionnées séparément ou ensemble dans un même article de conditionnement ou dans au moins deux articles de conditionnement séparés.

De préférence, ces compositions sont conditionnées séparément et avantageusement, dans des articles de conditionnement séparés.

L'objet de la présente invention est donc en particulier un produit cosmétique de maquillage se présentant sous la forme d'un rouge à lèvres, d'un fond de teint, d'un mascara, d'un fard à joues ou à paupières, d'un rouge à lèvres, d'un vernis à ongle, d'un produit ayant notamment des propriétés de soin, d'un mascara, d'un eye-liner, d'un produit anti-cernes, ou d'un produit de maquillage du corps (du type tatouage) ou des cheveux.

L'invention a aussi pour objet un kit de maquillage contenant un produit cosmétique de maquillage tel que défini précédemment, dans lequel les différentes compositions sont conditionnées séparément et sont avantageusement accompagnées de moyens d'application appropriés. Ces moyens peuvent être des pinceaux, des brosses, des stylos, des crayons, des feutres, des plumes, des éponges, des tubes et /ou des embouts mousse.

La première composition du produit selon l'invention peut constituer une couche de base appliquée sur la matière kératinique et la seconde composition une couche de dessus. Il est toutefois possible d'appliquer sous la première couche une sous couche ayant la constitution ou non de la seconde couche.

Il est aussi possible de déposer une surcouche sur la seconde couche ayant une constitution identique ou non à celle de la première couche. De préférence, le maquillage obtenu est un maquillage bicouche.

La deuxième composition peut également constituer une couche de base appliquée sur la matière kératinique et la première composition une couche de dessus.

En particulier la couche de base est un rouge à lèvres, un fond de teint, un mascara, un brillant à lèvres, un eye liner, un vernis à ongles, un produit de soin pour les ongles, un produit de maquillage du corps, et la couche du dessus (ou « top coat ») un produit de soin ou de protection.

L'invention se rapporte aussi à un procédé de maquillage de la peau et/ou des lèvres et/ou des phanères consistant à appliquer sur la peau et/ou les lèvres et/ou les phanères un produit cosmétique tel que défini précédemment.

L'invention a encore pour objet un procédé cosmétique de soin ou de maquillage de la peau et/ou des lèvres et/ou des phanères d'être humain, consistant à appliquer sur la peau, les lèvres et/ou les phanères une première couche d'une première composition comprenant au moins une résine choisie parmi les colophanes, les dérivés de colophanes, les résines hydrocarbonées et leurs mélanges, ladite résine présentant une masse moléculaire moyenne en nombre inférieure ou égale à 10 000 g/mol, puis à appliquer, sur tout ou partie de la première couche, une seconde couche d'une seconde composition comprenant une huile de haute viscosité.

Le produit selon l'invention peut être appliqué sur la peau aussi bien du visage que du cuir chevelu et du corps, des lèvres, de l'intérieur des paupières inférieures, et des phanères comme les ongles, les cils, les cheveux, les sourcils,

voire les poils. La seconde composition peut former des motifs et peut être appliquée avec un stylo, crayon ou tout autre instrument (éponge, doigt, pinceau, brosse, plume). Ce maquillage peut aussi être appliqué sur les accessoires de maquillage comme les faux ongles, faux cils, perruques ou encore des pastilles ou des patchs adhérents sur la peau ou les lèvres (du type mouches).

L'invention a enfin pour objet l'utilisation d'un produit cosmétique comprenant une première et une seconde compositions, la première composition comprenant comprenant au moins une résine choisie parmi les colophanes, les dérivés de colophanes, les résines hydrocarbonées et leurs mélanges, ladite résine présentant une masse moléculaire moyenne en nombre inférieure ou égale à 10 000 g/mol, et la seconde composition comprenant une huile de haute viscosité, pour conférer à la peau et/ou les lèvres et/ou les phanères un rendu cosmétique confortable et/ou brillant et/ou non transfert et/ou non migrant et /ou de bonne tenue.

<u>Première composition</u>

La première composition selon l'invention comprend comprenant au moins une résine choisie parmi les colophanes, les dérivés de colophanes, les résines hydrocarbonées et leurs mélanges, ladite résine présentant une masse moléculaire moyenne en nombre inférieure ou égale à 10 000 g/mol.

La première composition présente avantageusement une tenue à l'huile supérieure ou égale à 50%.
Ladite composition est avantageusement apte à former un film présentant un dépôt dont la tenue à l'huile est supérieure ou égale à 50%, par exemple 55%, 60%, voir même 65%.

La tenue à l'huile peut être mesurée selon la méthode suivante.

On prépare trois lames de verre chacune recouverte d'une feuille de collagène comme suit.
A une température de 28°C, on prépare une feuille de collagène (Boyau artificiel naturin ep.0,06 mm, a. 0,10 mm, d. 120mm) de 5 cm par 10 cm de côté et on la met à conditionner au moins deux heures à 90% d'Humidité Relative (HR). On remet la feuille de collagène à l'air libre et on la fixe immédiatement fermement et entièrement sur une lame de verre 4,6 cm par 7,6 cm. On attache la feuille de collagène sur l'envers de la lame avec du scotch 3M. La surface du collagène doit être plane et exempte de pli. Chaque lame est laissée en conditions ambiantes pendant 24 heures, avant de réaliser le test.

On coupe un rectangle de 5 cm par 10 cm dans une assiette de Styrofoam (type Amoco Selectables Plastics DL Tableware) au moyen d'un couteau et d'une règle en suivant le contour d'une lame de verre. On applique le produit selon l'invention sur chaque lame de verre et sur le rectangle de Styrofoam comme suit. On applique la composition à l'aide d'un applicateur mécanique de 25μm (bar coater).
On laisse les plaques en conditions ambiantes pendant 24 heures.

On applique 3 gouttes d'huile d'olive (environ 0,075 g) étalées avec un pinceau sur chacune des trois lames recouvertes de collagène et de composition. On sèche l'excédent avec un papier Kimwipes et on laisse les lames 30 minutes à température ambiante. On coupe 3 disques de Styrofoam blanc de 4 cm de diamètre.
On attache fermement avec du double face le disque de Styrofoam blanc au bout d'une masse de 2kg et, en exerçant une pression de 175g/cm$^2$, on pose doucement le poids à la surface d'une plaque (côté produit) et on procède, en 3 à 5 secondes, à une rotation d'un tour et demi du poids sur lui-même et ce, en maintenant la pression initale. On remonte le poids et on récupère le disque de Styrofoam. La mesure est effectuée pour chaque lame de verre avec un disque de Styrofoam propre.

On mesure ensuite le pourcentage de réflectance :

- o du dépôt de produit appliqué sur l'échantillon rectangulaire de Styrofoam (référencé A),
- o du disque propre de Styrofoam blanc (référencé B)
- o du disque décollé du poids après avoir appliqué la pression sur la lame enduite de produit cosmétique (référencé C)

La réflectance est mesurée sur une gamme de longueur d'onde comprise entre 400 et 700nm à l'aide d'un analyseur spectral (ouverture 25 mm de diamètre) avec un illuminant D65/10degrés. On choisit la longueur d'onde du minimum de réflectance pour le disque 'taché'. A cette longueur d'onde, on calcule la tenue selon l'équation

$$100 * (1 - [(C-B)/(A-B)] )$$

La tenue à l'huile est égale à la moyenne des trois mesures.

**Résine**

La résine utilisée dans la composition selon l'invention a de préférence une masse moléculaire moyenne en nombre inférieure ou égale à 10 000 g/mol, notamment allant de 250 à 10 000 g/mol de préférence inférieure ou égale à 5 000 g/mol, notamment allant de 250 à 5 000 g/mol, mieux, inférieure ou égale à 2 000 notamment allant de 250 à 2 000 g/mol et encore mieux inférieure ou égale à 1 000 g/mol notamment allant de 250 à 1 000 g/mol.

On détermine les masses molaires moyennes en nombre (Mn) par chromatographie liquide par perméation de gel (solvant THF, courbe d'étalonnage établie avec des étalons de polystyrène linéaire, détecteur réfractométrique).

La résine de la composition selon l'invention est avantageusement une résine dite tackifiante. De telles résines sont notamment décrites dans le Handbook of Pressure Sensitive Adhesive, edited by Donatas Satas, 3rd ed., 1989, p. 609-619.

La résine de la composition selon l'invention peut être choisie parmi la colophane ou un de ses dérivés, les résines hydrocarbonées et leurs mélanges.

Par « hydrocarboné », on entend un composé comprenant majoritairement du carbone et de l'hydrogène, et éventuellement des hétéroatomes tels que l'oxygène, l'azote ou le soufre. Le composé hydrocarboné est de préférence constitué de carbone et d'hydrogène.

La colophane est un mélange comprenant majoritairement des acides organiques appelés acides de colophane (principalement des acides de type abiétique et de type pimarique).
Il existe trois types de colophane : la colophane ("gum rosin") obtenue par incision sur les arbres vivants, la colophane de bois ("wood rosin") qui est extraite des souches ou du bois de pins, et l'huile de tall ("tall oil rosin") qui est obtenue d'un sous-produit provenant de la production du papier.

Les dérivés de colophane peuvent être issus en particulier de la polymérisation, de l'hydrogénation et/ou de l'estérification (par exemple avec des alcools polyhydriques tels que l'ethylène glycol, le glycérol, le pentaérhytritol) des acides de colophanes. On peut citer par exemple les esters de colophanes commercialisés sous la référence FORAL 85, PENTALYN H et STAYBELITE ESTER 10 par la société HERCULES ; SYLVATAC 95 et ZONESTER 85 par la société ARIZONA CHEMICAL ou encore UNIREZ 3013 par la société UNION CAMP.

Les résines hydrocarbonées peuvent être choisies parmi les polymères oléfiniques de faible masse moléculaire qui peuvent être classifiées, selon le type de monomère, qu'elles comprennent, en les polymères indéniques, les résines de pentadiène, les résines des dimères du cyclopentadiène et les résines terpéniques.

Les polymères indéniques peuvent être choisis parmi les polymères issus de la polymérisation en proportion majoritaire de monomère indène et en proportion minoritaire de monomères choisis parmi le styrène, le méthylindène, le méthylstyrène et leurs mélanges. Ces polymères peuvent éventuellement être hydrogénés, et peuvent présenter un poids moléculaire allant de 200 à 1 500 g/mol.
Le polymère hydrocarboné indénique est de préférence un copolymère bloc obtenu à partir d'indène, et de styrène ou d'un dérivé du styrène.
Selon un mode de réalisation préféré, la résine est choisie parmi les résines indéniques, en particulier les copolymères indène/méthylstyrène/styrène hydrogéné commercialisées sous la dénomination « REGALITE » par la société Eastman Chemical, tels que la REGALITE R 1100, REGALITE R 1090, REGALITE R-7100, REGALITE R1010 HYDROCARBON RESIN, REGALITE R1125 HYDROCARBON RESIN.

Comme exemples de polymères indéniques, on peut citer ceux commercialisés sous la référence ESCOREZ 7105 par la société Exxon Chem., NEVCHEM 100 et NEVEX 100 par la société Neville Chem., NORSOLENE S105 par la société Sartomer, PICCO 6100 par la société Hercules et RESINALL par la société Resinall Corp.
Comme autre exemple de polymère indénique, on peut citer les résines de pentadiène et d'indène, qui sont issues de la polymérisation d'un mélange de monomères de pentadiène et d'indène tels que ceux décrits ci-dessus, comme par exemple les résines commercialisées sous la référence ESCOREZ 2101 par la société Exxon Chemicals., NEVPENE 9500 par la société Neville Chem., HERCOTAC 1148 par la société Hercules., NORSOLENE A 100 par la société Sartomer, WINGTACK 86, WINGTACK EXTRA et WINGTACK PLUS par la société Goodyear.

Les résines de pentadiène peuvent être choisies parmi celles issues de la polymérisation majoritairement du monomère 1,3-pentadiène (trans ou cis pipérylène) et de monomères minoritaires choisis parmi l'isoprène, le butène, le 2-méthyl-2-butène, le pentène, le 1,4-pentadiène et leurs mélanges. Ces résines peuvent présenter un poids moléculaire allant de 1 000 à 2 500 g/mol.
De telles résines du 1,3-pentadiène sont commercialisées par exemple sous les références PICCOTAC 95 par la société Eastman Chemical, ESCOREZ 1304 par la société Exxon Chemicals., NEVTAC 100 par la société Neville Chem. ou WINGTACK 95 par la société Goodyear.

Les résines des dimères du cyclopentanediène peuvent être choisies parmi celles issues de la polymérisation de premiers monomères choisis parmi l'indène et le styrène, et de deuxièmes monomères choisis parmi les dimères du cyclopentanediène tels que le dicyclopentadiène, le méthyldicyclopentadiène, les autres dimères du pentanediène, et leurs mélanges. Ces résines présentent généralement un poids moléculaire allant de 500 à 800 g/mol, telles que par

exemple celles commercialisées sous la référence BETAPRENE BR 100 par la société Arizona Chemical Co., NEVILLE LX-685-125 et NEVILLE LX-1000 par la société Neville Chem., PICCODIENE 2215 par la société Hercules, PETRO-REZ 200 par la société Lawter ou RESINALL 760 par la société Resinall Corp.

Les résines terpéniques peuvent être choisies parmi celles issues de la polymérisation d'au moins un monomère choisi parmi l'$\alpha$-pinène, le $\beta$-pinène, le limonène, et leurs mélanges. Ces résines peuvent présenter un poids moléculaire allant de 300 à 2 000 g/mol. De telles résines sont commercialisées par exemple sous la dénomination PICCOLYTE A115 et S125 par la société Hercules, ZONAREZ 7100 ou ZONATAC 105 LITE par la société ARIZONA Chem.

On peut également citer comme résine hydrocarbonée, certaines résines commercialisées sous la dénomination EASTOTAC C6-C20 POLYOLEFINE par la société Eastman Chemical Co., sous la référence ESCOREZ 5300 par la société Exxon Chemicals ou encore les résines NEVILLAC HARD ou NEVROZ proposées par la société Neville Chem., les résines PICCOFYN A-100, PICCOTEX 100 ou PICCOVAR AP25 proposées par la société Hercules ou la résine SP-553 proposée par société Schenectady Chemical Co.

Un des objets de la présente invention est une composition cosmétique de maquillage des lèvres comprenant au moins un polymère hydrocarboné indénique de masse moléculaire moyenne en nombre inférieure ou égale à 10 000 g/mol. Cette composition est avantageusement apte à former un film présentant une tenue à l'huile supérieure ou égale à 50 %. Dans ce mode de réalisation, le polymère indénique peut être un des polymères indéniques décrits précédemment. Le polymère hydrocarboné indénique est de préférence un copolymère bloc obtenu à partir d'indène, et de styrène ou d'un dérivé du styrène.

La résine peut être présente dans la composition selon l'invention en une teneur allant de 0,1 à 40% en poids par rapport au poids total de la composition, de préférence de 0,5 à 30% en poids et mieux de 1 à 20% en poids, par exemple de 2 à 15% en poids.

La résine peut être dotée d'au moins une température de transition vitreuse de préférence supérieure ou égale à 20°C, de préférence supérieure ou égale à 30°C, de préférence de l'ordre de 40°C. La température de transition vitreuse est avantageusement comprise entre 20°C et 300°C, par exemple entre 30°C et 100°C.

Les températures de transition vitreuse indiquées dans la présente demande peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_{i} (\varpi_i / Tg_i) \ ,$$

$\omega_i$ étant la fraction massique du monomère i dans la séquence considerée et $Tg_i$ étant la température de transition vitreuse de l'homopolymère du monomère i.

La mesure de la température de transition vitreuse (Tg) peut être effectuée selon la norme ASTM D3418-97, par analyse enthalpique différentielle (DSC "Differential Scanning Calorimetry") sur calorimètre, sur une plage de température comprise entre -100°C et +150°C à une vitesse de chauffe de 10°C/min dans des creusets en aluminium de 150 $\mu$l.

## Phase grasse liquide

La composition selon l'invention peut comprendre une phase grasse liquide à température ambiante (25°C) et pression atmosphérique (760 mm de Hg), composée d'un ou plusieurs corps gras non aqueux liquides à température ambiante, appelés aussi huiles ou solvants organiques, compatibles entre eux.

L'huile peut être choisie parmi les huiles volatiles et/ou les huiles non volatiles, et leurs mélanges.

Par "huile volatile", on entend au sens de l'invention une huile susceptible de s'évaporer au contact de la peau ou de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg).

Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.
Comme huiles volatiles, on peut aussi utiliser les silicones volatiles, comme par exemple les huiles de silicones linéaires ou cycliques volatiles, notamment celles ayant une viscosité ≤ 8 centistokes ($8\ 10^{-6}\ m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.
On peut également citer les huiles linéaires alkyltrisiloxanes volatiles de formule générale (I) :

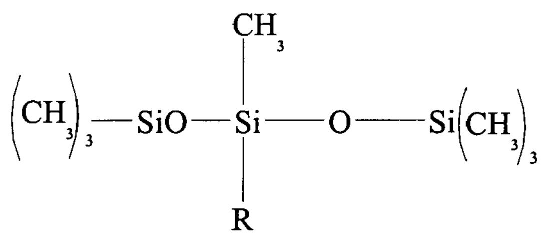

où R représente un groupe alkyle comprenant de 2 à 4 atomes de carbone et dont un ou plusieurs atomes d'hydrogène peuvent être substitués par un atome de fluor ou de chlore.
Parmi les huiles de formule générale (I), on peut citer :

le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

On peut également utiliser des solvants volatils fluorés tels que le nonafluorométhoxybutane ou le perfluorométhylcyclopentane.
La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :

- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;

- les acides gras supérieurs tels que l'acide oléique, l'acide linoléique, l'acide linolénique ;
- et leurs mélanges.

Selon un mode de réalisation de la présente invention, la composition contient une huile polaire, par exemple un alcool choisi parmi les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Selon un mode de réalisation, la phase grasse contient une huile ester. Cette huile ester peut être choisie parmi les esters des acides monocarboxyliques avec les monoalcools et polyalcools.

Avantageusement, ledit ester répond à la formule (I) suivante :

$$R_1\text{-CO-O-}R_2 \qquad (I)$$

où $R_1$ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué. $R_2$ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

Par « éventuellement substitué », on entend que $R_1$ et ou $R_2$ peuvent porter un ou plusieurs substituants choisis, par exemple, parmi les groupements comprenant un ou plusieurs hétéroatomes choisi parmi O, N et S, tels que amino, amine, alcoxy, hydroxyle.

De préférence, le nombre total d'atomes de carbone de $R_1 + R_2$ est $\geq 9$.

$R_1$ peut représenter le reste d'un acide gras, de préférence supérieur, linéaire ou, de préférence ramifié comprenant de 1 à 40 et mieux de 7 à 19 atomes de carbone et $R_2$ peut représenter une chaîne hydrocarbonée linéaire ou de préférence ramifiée contenant de 1 à 40, de préférence de 3 à 30 et mieux de 3 à 20 atomes de carbone. De nouveau, de préférence, le nombre d'atomes de carbone de $R_1 + R_2 \geq 9$.

Des exemples des groupes $R_1$ sont ceux dérivés des acides gras choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, éléostéarique, arachidonique, érucique, et de leurs mélanges.

Des exemples d'esters sont, par exemple, l'huile de purcellin (octanoate de cétostéaryle), l'isononanoate d'isononyle, le myristate d'isopropyle, le palmitate d'éthyl-2-hexyle, le stéarate d'octyl 2-dodécyle, l'érucate d'octyl 2-dodécyle, l'isostéarate d'isostéaryle, et les heptanoates, octanoates, décanoates ou ricinoléates d'alcools ou de polyalcools, par exemple d'alcools gras.

Avantageusement, les esters sont choisis parmi les composés de la formule (I) ci-dessus, dans laquelle $R_1$ représente un groupe alkyle linéaire ou ramifié non substitué, comprenant éventuellement une ou plusieurs double liaisons éthyléniques, de 1 à 40 atomes de carbone, de préférence de 7 à 19 atomes de carbone, et $R_2$ représente un groupe alkyle linéaire ou ramifié non substitué, comprenant éventuellement une ou plusieurs double liaisons éthyléniques, de 1 à 40 atomes de carbone, de préférence de 3 à 30 atomes de carbone, et mieux de 3 à 20 atomes de carbone.

De préférence, $R_1$ est un groupe alkyle ramifié non substitué de 4 à 14 atomes de carbone, de préférence de 8 à 10 atomes de carbone et $R_2$ est un groupe alkyle ramifié non substitué de 5 à 15 atomes de carbone, de préférence de 9 à 11 atomes de carbone. De préférence dans la formule (I), $R_1$-CO- et $R_2$ ont le même nombre d'atomes de carbone et dérivent du même radical, de préférence alkyle ramifié non substitué, par exemple isononyle, c'est-à-dire que avantageusement la molécule d'huile ester est symétrique.

L'huile ester sera choisie, de préférence, parmi les composés suivants :

- l'isononanoate d'isononyle,
- l'octanoate de cétostéaryle,
- le myristate d'isopropyle,
- le palmitate d'éthyl-2-hexyle,

- le stéarate d'octyl 2-dodécyle,
- l'érucate d'octyl 2-dodécyle,
- l'isostéarate d'isostéaryle.

La phase grasse liquide peut représenter de 0,5 à 90 % en poids par rapport au poids total de la composition, de préférence de 1 à 60 % et de façon encore plus préférée de 2 à 40 % en poids.

**Copolymère bloc hydrocarboné**

Selon un mode de réalisation de l'invention, la composition contient outre la résine, un copolymère séquencé hydrocarboné appelé également copolymère bloc, de préférence un copolymère séquencé soluble dans une phase grasse liquide telle que définie précédemment.

Le copolymère peut présenter au moins un bloc dont la température de transition vitreuse, est de préférence inférieure à 20°C, de préférence inférieure ou égale à 0°C, de préférence inférieure ou égale à -20°C, de préférence encore inférieure ou égale à -40°C. La température de transition vitreuse dudit bloc peut être comprise entre -150°C et 20°C, notamment entre -100°C et 0°C.

Dans ce cas, lorsque la résine est dotée d'au moins une température de transition vitreuse, l'écart entre les températures de transition vitreuse de la résine et du copolymère est généralement supérieur à 20°C, de préférence supérieur à 40°C, et mieux supérieur à 60°C.

Lorsque la résine est dotée d'au moins une température de transition vitreuse, le copolymère séquencé est avantageusement un plastifiant de la résine décrite précédemment. On entend par plastifiant de la résine, un composé, qui associé en quantité suffisante à la résine, abaisse la température de transition vitreuse de la résine telle que définie précédemment. Le composé plastifiant abaisse notamment la température de transition vitreuse du polymère d'au moins 2, 3 ou 4 °C, de préférence de 5°C à 20°C. Dans un mode de réalisation préféré, le composé plastifiant abaisse notamment la température de transition vitreuse du polymère d'au moins 2, 3 ou 4 °C, de préférence de 5°C à 20°C.

Le copolymère bloc peut être choisi parmi les copolymères séquencés, éventuellement hydrogénés, dibloc, tribloc, multi-bloc ou radiaux, et leurs mélanges.

On préfère notamment les copolymères séquencés comprenant au moins un bloc styrène et au moins un bloc comprenant des motifs choisis parmi le butadiène, l'éthylène, le propylène, le butylène, l'isoprène ou un de leurs mélanges.

Comme copolymère dibloc, on peut citer les copolymères de styrène/éthylène-propylène (constitués d'un bloc styrène et d'un bloc obtenu à partir d'éthylène et de propylène), les copolymères de styrène/éthylène-butylène, les copolymères styrène/butadiène, les copolymères styrène/isoprène. De tels copolymères sont notamment vendus sous la dénomination Kraton® G1701 E par la société Kraton Polymers.

Comme copolymère tribloc, on peut citer les copolymères de styrène/éthylène-propylène/styrène, les copolymères de styrène/éthylène-butylène/styrène, les copolymères de styrène/éthylène-butadiène/styrène, les copolymères de styrène/isoprène/styrène, les copolymères de styrène/butadiène/styrène. Des polymères triblocs sont notamment vendus sous les dénominations Kraton® G1650, Kraton® G1652, Kraton® D1101, Kraton® D1102, Kraton® D1160 par la société Kraton Polymers.

On peut notamment utiliser comme copolymère bloc, le mélange d'un copolymère dibloc et d'un copolymère tribloc. Dans ce mode de réalisation, le copolymère dibloc et le copolymère tribloc sont avantageusement choisis parmi les copolymères séquencés comprenant au moins un bloc styrène et au moins un bloc comprenant des motifs choisis parmi le butadiène, l'éthylène, le propylène, le butylène, l'isoprène.

On peut citer à titre d'exemple le produit commercialisé sous la référence Kraton G 1657 M, qui est un mélange de copolymère dibloc styrène/éthylène-butylène, et de copolymère tribloc styrène/éthylène-butylène/styrène dans les proportions 30/70, la température de transition vitreuse du bloc éthylène-butylène étant égale à -60°C environ.

On peut également utiliser un mélange de copolymère hydrogéné tribloc styrène/butylène-éthylène/styrène et de polymère étoile hydrogéné éthylène/propylène/styrène. De tels mélanges sont par exemple vendus par la société PENRECO sous les dénominations commerciales VERSAGEL® M5960 et VERSAGEL® M5670.

Le copolymère bloc hydrocarboné peut être présent en une quantité comprise entre 0,1 et 25% en poids du poids de la composition, notamment de 0,5 à 15 % en poids.

Le ratio massique entre la résine hydrocarbonée et le copolymère bloc hydrocarboné est de préférence compris entre 80/20 et 40/60, notamment entre 75/25 et 50/50.

La composition peut comprendre une phase aqueuse, qui comprend de l'eau et/ou au moins un solvant hydrosoluble.

Par "solvant hydrosoluble", on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions selon l'invention, on peut citer notamment

les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en $C_3$ et $C_4$ et les aldéhydes en $C_2$-$C_4$.
La phase aqueuse (eau et éventuellement le solvant miscible à l'eau) peut être présente, en une teneur allant de 5 % à 95 % en poids, par rapport au poids total de la composition, de préférence allant de 10 % à 80 % en poids, et préférentiellement allant de 15 % à 60 % en poids.
La composition selon l'invention peut contenir des agents tensioactifs émulsionnants présents notamment en une proportion allant de 0,1 à 30 % en poids par rapport au poids total de la composition, mieux de 1 à 15 % et mieux de 2 à 10 %.
La composition selon l'invention peut comprendre au moins un agent structurant de la phase huileuse choisi parmi

- les polymères semi-cristallins, décrits par exemple dans le document EP 1396259,
- les gélifiants lipophiles minéraux, comme les argiles éventuellement modifiées comme les hectorites modifiées par un chlorure d'ammonium d'acide gras en $C_{10}$ à $C_{22}$, comme l'hectorite modifiée par du chlorure de di-stéaryl di-méthyl ammonium telle que, par exemple, celle commercialisée sous la dénomination de Bentone 38V® par la société ELEMENTIS. On peut également citer la silice pyrogénée éventuellement traitée hydrophobe en surface dont la taille des particules est inférieure à 1 $\mu$m.
- les organogélateurs moléculaires notamment ceux décrits dans le document "Specialist Surfactants", édité par D. Robb de 1997, p.209-263, chapitre 8 de P. Terech, les demandes européennes EP-A-1068854 et EP-A-1086945 ou encore dans la demande WO-A-02/47031.
- les organopolysiloxanes élastomériques partiellement ou totalement réticulés, de structure tridimensionnelle, comme ceux commercialisés sous les dénominations de KSG6®, KSG16® et de KSG18® par la société SHIN-ETSU
- les polycondensats de type polyamide comprenant au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL ;
- les polyamides siliconés du type polyorganosiloxane tels que ceux décrits dans les documents US-A-5 874 069, US-A-5,919,441, US-A-6,051,216 et US-A-5,981,680 comme par exemple ceux commercialisés sous la référence Dow Corning 2-8179 Gellant par la société DOW CORNING;
- les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges ;

La composition selon l'invention comprend avantageusement au moins une cire.
La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.
En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.
En particulier, les cires convenant à l'invention peuvent présenter un point de fusion supérieur ou égal à 45 °C, et en particulier supérieur ou égal à 55°C.
Les cires susceptibles d'être utilisées dans les compositions selon l'invention sont choisies parmi les cires, solides, à température ambiante d'origine animale, végétale, minérale ou de synthèse et leurs mélanges.
A titre illustratif des cires convenant à l'invention, on peut notamment citer les cires hydrocarbonées comme la cire d'abeilles, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.
On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.
On peut encore citer les cires de silicone, les cires fluorées.
La composition selon l'invention peut comprendre une teneur en cires allant de 5 à 20 % en poids par rapport au poids total de la composition, en particulier elle peut en contenir de 7 à 15%.

La composition selon l'invention peut également comprendre au moins un corps gras pâteux.

Par corps gras pâteux, on entend un composé lipophile comportant à la température de 23°C une fraction liquide et une fraction solide. Par corps gras pâteux, on entend également le polylaurate de vinyle.

Ledit composé pâteux a de préférence une dureté à 20°C allant de 0,001 à 0,5 MPa, de préférence de 0,002 à 0,4 MPa. La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum.

La fraction liquide du composé pâteux mesurée à 23°C représente de préférence 9 à 97% en poids du composé. Cette fraction liquide à 23°C représente de préférence entre 15 et 85%, de préférence encore entre 40 et 85% en poids. La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée eu J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

## Seconde composition

Le produit cosmétique selon l'invention comprend une seconde composition contenant un milieu cosmétiquement acceptable.

La seconde composition est avantageusement choisie de telle manière qu'elle améliore au moins une propriété cosmétique de la première composition, lorsque celle-ci est appliquée seule sur ladite matière kératinique. La deuxième composition peut notamment améliorer le confort de la première composition, notamment diminuer son caractère collant.

La seconde composition peut également être choisie de manière à ce que le produit, une fois appliqué sur les matières kératiniques, présente des propriétés de brillance satisfaisantes.

La première composition et/ou la seconde composition, peuvent être choisies de manière à ce que le produit, une fois appliqué sur les matières kératiniques, présente des propriétés de brillance satisfaisantes.

### *Brillance*

Avantageusement, le produit selon l'invention peut posséder une brillance supérieure ou égale à 5, en particulier supérieure ou égale à 10, notamment supérieure ou égale à 15, plus particulièrement supérieure ou égale à 20, notamment supérieure ou égale à 25, voire de l'ordre de 30.

Par "brillance", on désigne la brillance telle qu'elle peut être mesurée par la méthode suivante, en utilisant un appareillage de type gonio-réflectomètre comme par exemple le GRM-2000, commercialisé par la société MICROMODULES.

Les paramètres retenus pour cet appareillage sont les suivants :

- angle d'éclairage : 120°
- angle de détection : 60°
- angle de début : 50°
- angle de fin : 95°

On constitue un support de type mousse de forme rectangulaire, de dimensions 40 x 70 mm à partir d'une mousse de couleur brique, par exemple une mousse de néoprène de 3 mm d'épaisseur et qui possède une face adhésive, notamment une mousse connue sous la référence commerciale RE40x70 EP3 commercialisée par la société Joint Technique Lyonnais Ind.

On fixe, sur la face opposée à la face adhésive de ce support, un sparadrap transparent commercialisé par la société 3M® sous la référence commerciale BLENDERM® FH 5000-55113, présentant une qualité d'usage telle que l'application d'un rouge à lèvres sur ce revêtement est similaire à celle que l'on réalise sur les lèvres.

Le support mousse revêtu du sparadrap transparent est ensuite fixé, par collage, à l'aide de sa face à adhésive à une plaque métallique de dimension 40 x 70 mm. L'ensemble constitué par le support collé sur la plaque métallique forme une éprouvette.

L'opérateur réalise au total 5 éprouvettes identiques à celle décrite ci-dessus.

On va maintenant décrire un mode de mise en oeuvre du procédé d'évaluation de la brillance.

L'opérateur dispose l'éprouvette sur une plaque chauffante réglée à température de 38,5 °C, par exemple une plaque chauffante de type N81076 commercialisée par la société FISHER BIOBLOCK, et attend que la face du support portant le revêtement adhésif atteigne une température de 33 °C ± 1 °C.

Une fois que le support est à la température voulue, l'opérateur applique un film d'une épaisseur d'environ 15 µm de la première composition sur le revêtement adhésif. Il laisse la première composition sécher, si elle contient des huiles ou des solvants volatiles. L'Opérateur applique ensuite un film d'une épaisseur d'environ 15 µm de la deuxième composition sur le film de première composition sec.

Le produit cosmétique, qui est par exemple un rouge à lèvres, a été stocké à 24 °C ± 2 °C.

Le geste effectué par l'opérateur pour déposer le film de produit consiste en un aller/retour, de manière à obtenir un dépôt homogène. L'application du produit sur le support est effectuée de préférence de manière à être la plus représentative possible des conditions réelles d'application du produit. Le même produit à tester est appliqué de façon identique sur les cinq mêmes éprouvettes préparées précédemment.

On laisse sécher le film du produit, l'éprouvette étant placée sur la plaque chauffante, de telle sorte que le support reste à 33 °C ± 1 °C pendant 10 minutes.

On mesure la brillance du film du produit pour chacune des cinq éprouvettes.

A partir des valeurs mesurées, on établit la valeur moyenne de la brillance selon les conventions suivantes :

$$\overline{Brillance} = \frac{1}{N}\sum_i Brillance_i$$

$$\text{écart-type : } \sigma_{Brillance} = \sqrt{\frac{N\sum_i Brillance_i^2 - (\sum_i Brillance_i)^2}{N(N-1)}}$$

intervalle de confiance à 95 % : Brillance ± 1,96 $\sqrt{\frac{\sigma_{Brillance}}{N}}$

où N désigne le nombre de mesures, c'est-à-dire 5 dans le cas d'espèce.

Le milieu cosmétiquement acceptable de la seconde composition comprend de préférence un corps gras non volatile à température ambiante et pression atmosphérique.

Par « corps gras non volatile », on entend un corps gras liquide ou solide ayant en particulier une pression de vapeur à une température ambiante de 25°C et une pression atmosphérique de 1 bar, non nulle inférieure à 0,02 mm de Hg (2,66 Pa) et mieux inférieure à $10^{-3}$ mm de Hg (0,13 Pa).

Le corps gras peut être hydrocarboné, siliconé, ou fluoré, ou être constitué de l'un de leurs mélanges. Le corps gras peut être un solide ou une huile visqueuse.

Par "hydrocarboné", on entend un composé comprenant majoritairement des atomes de carbone et des atomes d'hydrogène et en particulier des chaînes alkyle ou alcényle comme les alcanes ou alcènes mais aussi les huiles à chaîne alkyle ou alcényle comportant un ou des groupements éther, ester ou acide carboxylique.

Par « siliconé », on entend un composé comprenant des atomes de silicium.

Par « fluoré », on entend un corps gras un composé comprenant majoritairement des atomes de carbone, de

fluor et d'hydrogène.

La seconde composition contient avantageusement un phase grasse liquide non volatile. Celle-ci peut représenter 1 à 100 %, de préférence de 5 à 95 %, mieux de 20 à 80 % et mieux encore, de 40 à 80 % du poids total de la seconde composition.

Par phase grasse liquide, on entend une phase comprenant un ou plusieurs corps gras liquides à une température ambiante de 25°C et une pression atmosphérique de 1 bar.

Selon un mode de réalisation, le corps gras non volatile est une huile non volatile visqueuse.

Par huile visqueuse, on entend une huile dont la viscosité à 25°C est avantageusement supérieure ou égale à 100 cSt, notamment supérieure ou égale à 500 cSt, voir même supérieure ou égale à 1000 cSt. L'huile visqueuse présente avantageusement une masse moléculaire supérieure ou égale à 600 g/mol, par exemple supérieure ou égale à 700, voir 800, voir même 900 g/mol.

L'huile visqueuse a notamment une viscosité à 25°C comprise entre 100 et 5 000 000 cSt, notamment entre 500 et 1 000 000 cSt, par exemple entre 1 000 et 700 000 cSt, par exemple encore entre 10 000 cSt et 500 000 cSt, par exemple égale à 350 cSt, 60 000 cSt ou 300 000 cSt.

L'huile visqueuse présente avantageusement une masse moléculaire comprise entre 600 et 2 500 000 g/mol, par exemple entre 700 et 2 000 000 g/mol, par exemple encore entre 1 000 et 500 000 g/mol, par exemple encore entre 10 000 et 300 000 g/mol.

La viscosité dynamique à 25 °C de l'huile visqueuse peut être mesurée avec un viscosimètre rotatif METTLER RM 180, en prenant en compte la densité de l'huile pour effectuer la conversion en cSt.

La viscosité dynamique de l'huile peut être mesurée avec un viscosimètre de type METTLER RM 180. L'appareil METTLER RM 180 (Rhéomat) peut être équipé de différents mobiles en fonction de l'ordre de grandeur de la viscosité que l'on veut mesurer. Pour une viscosité comprise entre 0,18 et 4,02 Pa.s, on équipe l'appareil d'un mobile 3. Pour une viscosité comprise entre 1 et 24 Pa.s, on équipe l'appareil d'un mobile 4, et pour une viscosité comprise entre 8 et 122 Pa.s, on équipe l'appareil d'un mobile 5. La viscosité est lue sur l'appareil en unités de déviation (UD). On se reporte ensuite à des abaques fournies avec l'appareil de mesure pour obtenir la valeur correspondante en poises, puis effectuer la conversion en stokes.

La vitesse de rotation du mobile est de 200 tours/min.

A partir du moment où le mobile est mis en rotation, à une vitesse de rotation constante imposée (en l'espèce 200 tours/min), la valeur de viscosité de l'huile peut varier au cours du temps. Des mesures sont prises à intervalle de temps réguliers jusqu'à ce qu'elles deviennent constantes. La valeur de la viscosité devenue constante au cours du temps est la valeur retenue comme étant la valeur de la viscosité dynamique de l'huile visqueuse.

La proportion du corps gras non volatile dans la deuxième composition peut être comprise entre 2 et 100% en poids du poids de la deuxième composition, par exemple de 5 à 80%, voir de 10 à 70%, voir même de 20 à 40% en poids du poids de la composition.

L'huile visqueuse peut être hydrocarbonée, fluorée ou siliconée.

L'huile visqueuse peut être choisie parmi les huiles décrites précédemment dans le paragraphe concernant la phase grasse liquide de la première composition.

L'huile visqueuse notamment être choisie parmi les polybutènes et le malate de di-isostéaryle.

L'huile visqueuse peut être siliconée, et être par exemple une huile siliconée phénylée, une huile siliconée comprenant des atomes de fluor, ou une huile polydiméthylsiloxane,.

L'huile visqueuse siliconée non volatile peut être choisie parmi les polyalkylsiloxanes, les polyarylsiloxanes, les polyalkylarylsiloxanes et leurs mélanges.

L'huile siliconée non volatile peut être choisie parmi

- les polydiméthylsiloxanes (PDMS) non volatils, linéaires ou ramifiés ;
- les polydiméthylsiloxanes comportant des groupements alkyle, alcoxy ou phényle, pendant

ou en bout de chaîne siliconée, groupements ayant de 2 à 24 atomes de carbone ;

- les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les polyalkylsiloxanes selon l'invention incluent les polydiméthylsiloxanes, les copolymères (polydiméthylsiloxane)(méthylvinylsiloxane), les poly(diméthylsiloxane)(diphényl)siloxanes, les copolymères poly(diméthylsiloxane)(diphényl)(méthylvinylsiloxane), et leurs mélanges

L'huile siliconée fluorée peut correspondre à la formule :

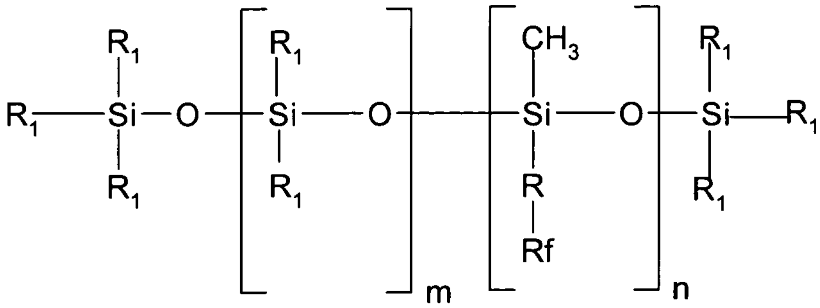

dans laquelle :

- R représente un groupement alkylényle linéaire ou ramifié, ayant 1 à 6 atomes de carbone, de préférence un groupement divalent méthylényle, éthylényle, propylényle ou butylényle,
- Rf représente un radical fluoroalkyle, notamment un radical perfluoroalkyle, ayant 1 à 9 atomes de carbone, de préférence 1 à 4 atomes de carbone,
- $R_1$ représentent, indépendamment l'un de l'autre, un radical alkyle en $C_1$-$C_{20}$, de préférence en $C_1$-$C_4$, un radical hydroxyle, ou un radical phényle,
- m est choisi de 0 à 150, de préférence de 20 à 100, et
- n est choisi de 1 à 300, de préférence de 1 à 100.

Le corps gras non volatile peut être un polymère siliconé solide ou liquide.

Selon un mode de réalisation, le polymère siliconé a une masse moléculaire en poids supérieure ou égale à 200 000 g/mol, de préférence comprise entre 200 000 et 2 500 000, de préférence entre 200 000 et 2 000 000 g/mol.

La viscosité de ce polymère peut être comprise entre 10 000 et 5 000 000 cSt, de préférence entre 100 000 et 1 000 000 cSt, de préférence encore entre 300 000 et 700 000 cSt.

Le polymère siliconé est avantageusement un polymère non greffé, c'est à dire un polymère obtenu par polymérisation d'au moins un monomère, sans réaction subséquente des chaînes latérales avec un autre composé chimique. Le polymère est de préférence choisi parmi les diméthiconols, les fluorosilicones, les diméthicones et leurs mélanges. Le polymère est de préférence un homopolymère.

En particulier, on peut utiliser un polymère répondant à la formule (A):

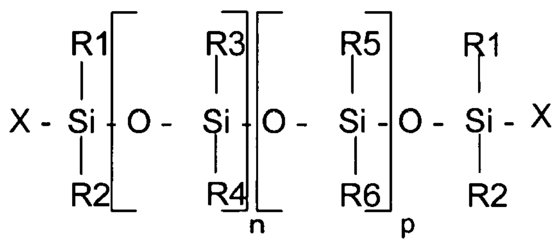

(A)

dans laquelle :

$R_1$, $R_2$, $R_5$ et $R_6$ sont, ensemble ou séparément, un radical alkyle ayant 1 à 6 atomes de carbone, éventuellement substitué par au moins un atome de fluor,

$R_3$ et $R_4$ sont, ensemble ou séparément, un radical alkyle ayant de 1 à 6 atomes de carbone ou un radical aryle,

X est un radical alkyle ayant de 1 à 6 atomes de carbone, un radical hydroxyle, un radical vinyle, allyle ou un radical alkoxyle ayant de 1 à 6 atomes de carbone,

n et p étant choisis de manière à ce que le composé siliconé ait une masse moléculaire en poids supérieure ou égale à 600 g/mol.

Les polymères de formule (A) tels $R_1$ à $R_6$ représentent un groupement méthyle et le substituant X représente

un groupement hydroxyle sont des diméthiconols. On cite par exemple les polymères de formule (A) tels que p=0 et n est compris entre 2 000 et 40 000, de préférence entre 3 000 et 30 000. On peut également citer les polymères ayant une masse moléculaire comprise entre 1 500 000 et 2 000 000 g/mol.

Selon un mode de mise en oeuvre, le polymère de haut poids moléculaire est la diméthiconol commercialisée par Dow Corning dans une polydiméthylsiloxane (5 cSt) sous la référence D2-9085, la viscosité du mélange étant égale à 1 550 cSt, ou la diméthiconol commercialisée par Dow Corning dans une polydiméthylsiloxane sous la référence DC 1503. On préfère également la diméthiconol (de poids moléculaire égal à 1 770 000 g/mol) commercialisée par Dow Corning sous la référence Q2-1403 ou Q2-1401, la viscosité du mélange étant égale à 4 000 cSt.

Comme polymère siliconé utilisable selon l'invention, on peut citer ceux pour lesquels :

- les substituants $R_1$ à $R_6$ et X représentent un groupement méthyle, comme celle vendue sous la dénomination SE30 par la société Général Electric, et celle vendue sous la dénomination AK 500000 par la société Waker,
- les substituants $R_1$ à $R_6$ et X représentent un groupement méthyle, p et n sont tels que le poids moléculaire est de 250 000 g/mol, comme celle vendue sous la dénomination Silbione 70047 V par la société Rhodia,
- les substituants $R_1$ à $R_6$ représentent un groupement méthyle, le substituant X représente un groupement hydroxyle, comme celle vendue sous la dénomination Q2-1401 ou Q2-1403 par la société Dow Corning,
- les substituants $R_1$, $R_2$, $R_5$, $R_6$ et X représentent un groupement méthyle, les substituants $R_3$ et $R_4$ représentent un groupement aryle, n et p sont tels que le poids moléculaire du polymère soit de 600 000 g/mol, comme celle vendue sous la dénomination 761 par la société Rhône-Poulenc.

Le polymère siliconé peut être introduit dans la deuxième composition sous la forme d'un mélange avec une silicone liquide, la viscosité de ladite silicone liquide étant comprise entre 0,5 et 10 000 cSt, de préférence entre 0,5 et 500 cSt, de préférence encore entre 1 et 10 cSt.

La silicone fluide peut être choisie parmi les polyalkylsiloxanes, les polyarylsiloxanes, les polyalkylarylsiloxanes et leurs mélanges. La silicone liquide peut être une silicone volatile telle qu'une polydiméthylsiloxane cyclique comprenant 3 à 7 motifs $-(CH_3)_2SiO-$.

La silicone liquide peut également être une silicone non volatile polydiméthylsiloxane, notamment de viscosité comprise entre 0,5 et 10 000 cSt, de préférence encore de l'ordre de 5 cSt, par exemple la silicone commercialisée sous la référence DC 200 par Dow Corning.

Les viscosités des silicones fluides sont mesurées, dans l'ensemble de la présente demande selon la norme ASTM D-445. La proportion du polymère de haut poids moléculaire dans le mélange polymère de haut poids moléculaire/silicone liquide est de préférence compris entre 10/90 et 20/80. La viscosité du mélange polymère de haut poids moléculaire/ silicone liquide est de préférence comprise entre 1 000 et 10 000 cSt.

Les diméthicones de haut poids moléculaire selon l'invention incluent les diméthicones décrites dans le brevet US 4 152 416. Elles sont par exemple commercialisées sous les références SE30, SE33, SE 54 et SE 76.

Les diméthicones selon l'invention sont par exemple des composés de formule (III) telle que $R_1$ à $R_6$ et X sont des méthyles et p=0. Le poids moléculaire de ces polymères est de préférence compris entre 200 000 et 300 000, de préférence entre 240 000 et 260 000 g/mol.

Les diméthicones selon l'invention incluent les polydiméthylsiloxanes, les copolymères (polydiméthylsiloxane)(méthylvinylsiloxane), les copolymères poly(diméthylsiloxane)(diphényl)(méthylvinylsiloxane), et leurs mélanges

Les fluorosilicones de haut poids moléculaire selon l'invention ont de préférence un poids moléculaire compris entre 200 000 et 300 000, de préférence entre 240 000 et 260 000 g/mol.

La deuxième composition peut contenir le mélange d'une polydiméthylsiloxane de masse moléculaire comprise entre 200 000 et 300 000 g/mol et d'une polydiméthylsiloxane de masse moléculaire comprise entre 400 et 1 000 g/mol. Dans ce cas, la proportion massique composé siliconé de haut poids moléculaire/composé siliconé de bas poids moléculaire est de préférence comprise entre 20/80 et 60/40, de préférence entre 35/65 et 45/55.

La deuxième composition peut éventuellement contenir une huile peu visqueuse, de viscosité à 25°C inférieure à 500 cSt et/ou de masse moléculaire inférieure à 600 g/mol.

Cette huile peut être une des huiles décrites précédemment dans le paragraphe de la phase grasse liquide de la première composition.

Selon un mode de réalisation, la deuxième composition est transparente.

On entend par « composition transparente », une composition transparente à translucide, c'est-à-dire telle qu'elle transmet au moins 40 % de lumière à une longueur d'onde de 750 nm, et de préférence au moins 50 % de lumière.

La mesure de la transmission est effectuée avec un spectrophotomètre UV-visible Cary 300 Scan de la société Varian selon le protocole suivant :

On coule la composition au-dessus de sa température de fusion dans une cuve à spectrophotomètre à section carrée dont le côté a une longueur de 10 mm.

L'échantillon de la composition est ensuite refroidi pendant 24 heure à 35 °C puis maintenu dans une enceinte thermostatée à 20 °C pendant 24 heures.
On mesure ensuite la lumière transmise à travers l'échantillon de la composition au spectrophotomètre par balayage de longueurs d'onde allant de 700 nm à 800 nm, la mesure étant faite en mode transmission.
On détermine alors le pourcentage de lumière transmise à travers l'échantillon de la composition à la longueur d'onde de 750 nm.
Lorsque la seconde composition est transparente, elle contient avantageusement moins de 5% de pigments, de préférence moins de 2%, de préférence encore moins de 1 %.

Lorsque la première ou la deuxième composition est destinée à être appliquée sur les ongles, la composition contient de préférence un solvant choisi parmi:

- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- les composés hétérocycliques tels que le tétrahydrofuranne ;
- le carbonate de propylène, le 3-éthoxypropionate d'éthyle ;
- leurs mélanges.

On préfère en particulier l'acétate de méthyle, l'acétate d'isopropyle, l'acétate de méthoxypropyle, le lactate de butyle, l'acétone, la méthyléthylcétone, le diacétone alcool, la γ-butyrolactone, le tétrahydrofuranne, le carbonate de propylène, le 3-éthoxypropionate d'éthyle, le diméthylsulfoxide, et leurs mélanges.

La première et/ou la deuxième composition du produit cosmétique selon l'invention peut contenir un agent de coloration qui peut être choisi parmi les colorants hydrosolubles ou liposolubles, les pigments, les nacres et leurs mélanges.

Par « pigments », il faut comprendre des particules blanches ou colorées, minérales ou organiques, insolubles dans la phase organique liquide, destinées à colorer et/ou opacifier la première composition.
Par « nacres », il faut comprendre des particules irisées, notamment produites par certains mollusques dans leur coquille ou bien synthétisées, insolubles dans le milieu de la première composition.
Par « colorants », il faut comprendre des composés généralement organiques solubles dans les corps gras comme les huiles ou dans une phase hydroalcoolique.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Les pigments, les nacres ou les charges solides peuvent être dispersées dans la phase grasse liquide de la composition en présence d'un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans la composition de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy12-stéarique sont de préférence destinés à un milieu hydrocarboné ou fluoré, alors que les mélanges de diméthylsiloxane oxyéthylène/oxypropyléné sont de préférence destinés à un milieu siliconé.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi le mica recouvert de titane ou d'oxychlorure de bismuth, le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre au moins une charge, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la Société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La première et/ou la deuxième composition de l'invention peut, en outre, contenir un ou plusieurs actifs cosmétiques ou dermatologiques tels que ceux classiquement utilisés.

Comme actifs cosmétiques, dermatologiques, hygiéniques ou pharmaceutiques, utilisables dans la composition de l'invention, on peut citer les hydratants, vitamines, acides gras essentiels, sphingolipides, filtres solaires. Ces actifs sont utilisés en quantité habituelle pour l'homme de l'art et notamment à des concentrations de 0 à 20 % et notamment de 0,001 à 15 % du poids total de la première ou deuxième composition.

La composition peut comprendre, en outre, tout autre additif usuellement utilisé dans de telles compositions, tel que de l'eau, des antioxydants, des parfums, des conservateurs et des huiles essentielles.

Bien entendu l'homme du métier veillera à choisir ce ou ces éventuels composés complémentaires, et/ou leur quantité, de manière telle que les propriétés avantageuses de la composition ne soient pas, ou substantiellement pas, altérées par l'adjonction envisagée.

Les compositions du produit peuvent se présenter sous forme d'un produit coulé et par exemple sous la forme d'un stick ou bâton, ou sous la forme de coupelle utilisable par contact direct ou à l'éponge. En particulier, elles trouvent une application en tant que fond de teint coulé, fard à joues ou à paupières coulé, rouge à lèvres, base ou baume de soin pour les lèvres, produit anti-cernes, vernis à ongles. Elles peuvent aussi se présenter sous forme d'une pâte souple ou encore de gel, de crème plus ou moins fluide, ou de liquide conditionné dans un tube.

Les compositions du produit selon l'invention peuvent constituer notamment une composition cosmétique de soin pour le visage, pour le cou, pour les mains ou pour le corps (par exemple crème de soin, huile solaire, gel corporel),

une composition de maquillage (par exemple gel de maquillage, crème, stick) ou une composition de bronzage artificiel ou de protection de la peau.

Les compositions du produit selon l'invention peuvent se présenter sous la forme d'une composition de soin de la peau et/ou des phanères ou sous forme d'une composition de protection solaire, d'hygiène corporelle, notamment sous forme de déodorant. Elles se présentent alors notamment sous forme non colorée. Elles peuvent alors être utilisées comme base de soin pour la peau, les phanères ou les lèvres (baumes à lèvres, protégeant les lèvres du froid et/ou du soleil et/ou du vent, crème de soin pour la peau, les ongles ou les cheveux).

Par cosmétiquement acceptable, on entend au sens de l'invention une composition d'aspect, d'odeur et de toucher agréables.

Chaque composition du produit selon l'invention peut se présenter sous toute forme galénique normalement utilisée pour une application topique et notamment sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules, les vésicules étant situés à l'interface huile/eau, ou d'une poudre. Chaque composition peut être fluide ou solide.

Selon un mode de mise oeuvre, la première ou la seconde composition, ou les deux, sont à phase grasse continue et de préférence sous forme anhydre et peuvent contenir moins de 5% d'eau, et encore mieux moins de 1 % d'eau par rapport au poids total de la première ou seconde composition.

Chaque première et seconde composition peut avoir l'aspect d'une lotion, d'une crème, d'une pommade, d'une pâte souple, d'un onguent, d'un solide coulé ou moulé et notamment en stick ou en coupelle, ou encore de solide compacté.

Chaque composition peut être conditionnée séparément dans un même article de conditionnement par exemple dans un stylo bi-compartimenté, la composition de base étant délivrée par une extrémité du stylo et la composition du dessus étant délivrée par l'autre extrémité du stylo, chaque extrémité étant fermée notamment de façon étanche par un capuchon.

Alternativement, chacune des compositions peut être conditionnée dans un article de conditionnement différent.

De préférence, la composition qui est appliquée en première couche est sous forme liquide ou pâteuse, ce qui est hautement souhaitable dans le cas d'un rouge à lèvres ou d'un eyeliner.

Le produit selon l'invention peut être avantageusement utilisé pour le maquillage de la peau et/ou des lèvres et/ou des phanères selon la nature des ingrédients utilisés. En particulier, le produit de l'invention peut se présenter sous forme de fond de teint solide, de bâton ou pâte de rouge à lèvres, de produit anti-cernes, ou contours des yeux, d'eye liner, de mascara, d'ombre à paupières, de produit de maquillage du corps ou encore un produit de coloration de la peau.

Avantageusement, la deuxième composition présente des propriétés de soin, de brillance ou de transparence.

L'invention a encore pour objet un produit à lèvres, un vernis, un mascara, un fond de teint, un tatouage, un fard à joues ou à paupières comprenant une première et une seconde compositions telles que décrites précédemment.

Les compositions du produit de l'invention peuvent être obtenues par chauffage des différents constituants à la température de fusion des cires les plus élevées, puis coulage du mélange fondu dans un moule (coupelle ou doigt de gant). Elles peuvent aussi être obtenues par extrusion comme décrit dans la demande EP-A-0 667 146.

L'invention est illustrée plus en détail dans les exemples suivants. Les pourcentages sont des pourcentages en poids.

## Exemple 1 :

### Première composition : rouge à lèvres

On prépare le rouge à lèvres suivant selon l'invention:
Les proportions sont en grammes

| | |
|---|---|
| Copolymère styrène/méthyl styrène/indène hydrogéné (REGALITE R1100 de Eastmann) | 16 |
| Hydrogenated styrene/butdiene copolymer (Kraton 1657 M) * | 10 |
| Isododécane | Qsp 100 |
| Octyldodécanol | 3 ,1 |
| Pigments | 3,1 |

(suite)

| | |
|---|---|
| Nacres | 2 |

* Le Kraton 1657 M est un mélange de copolymère dibloc et de copolymère tribloc dans les proportions 70/30 comprenant des blocs styrène et des blocs éthylène-butylène.

On a mesuré la tenue à l'huile et la brillance de la composition selon les méthodes de mesure indiquées précédemment dans la description.
La tenue à l'huile est égale à 68%. La brillance moyenne d'un dépôt de composition de 50 microns mesurée à 20° est égale à 51 (± 1,5). La brillance moyenne d'un dépôt de composition de 50 microns mesurée à 60° est égale à 87 (± 1).

Mode opératoire:

1. On réalise un broyat pigmentaire des pigments dans la phase huileuse en effectuant 3 passages du mélange à la broyeuse tri cylindres.
2. On introduit dans un poêlon, le ou les copolymères et l'huile, on place le mélange sous agitation Rayneri à une température de 100°C.
3. quand on observe un mélange liquide transparent, on introduit le broyat et les nacres, on maintient place le mélange sous agitation Rayneri à une température de 100°C pendant 20 minutes
4. On réalise un qsp 100% avec l'huile
5. On coule la formule dans des pots étanches à l'isododécane.

**Deuxième composition : Gloss pour les lèvres**

| | |
|---|---|
| Polydiméthylsiloxane commercialisée sous la référence AK 300000 par Wacker (300 000 cSt) | 33% |
| Polydiméthylsiloxane commercialisée sous la référence DC 200 (350 cSt) par Dow Corning | 43% |
| Mélange de polydiméthylsiloxane alpha-oméga dihydroxylée et de polydiméthylsiloxane 5 cSt commercialisé sous la référence DC 2-9085 par Dow Corning | 14% |
| Pentacyclosiloxane | 10% |

**Revendications**

**1.** Produit cosmétique comprenant une première et une seconde compositions, la première composition contenant au moins une résine choisie parmi la colophane et ses dérivés, les résines hydrocarbonées et leurs mélanges, ladite résine présentant une masse moléculaire moyenne en nombre inférieure ou égale à 10 000 g/mol, et la seconde composition distincte de la première comprenant au moins un corps gras non volatile.

**2.** Produit selon la revendication 1, **caractérisé en ce que** la première composition est apte à former un film présentant une tenue à l'huile supérieure ou égale à 50%, voir même supérieure ou égale à 60%, et mieux encore supérieure ou égale à 65%.

**3.** Produit selon la revendication 1 ou 2, **caractérisé en ce que** la résine présente une masse moléculaire moyenne en nombre allant de 250 à 10 000 g/mol de préférence inférieur ou égal à 5 000 g/mol, notamment allant de 250 à 5 000 g/mol, mieux, inférieur ou égal à 2 000 notamment allant de 250 à 2 000 g/mol et encore mieux inférieur ou égal à 1 000 g/mol notamment allant de 250 à 1 000 g/mol.

**4.** Produit selon l'une des revendications précédentes, **caractérisé en ce que** les résines hydrocarbonées sont choisies parmi :

- les polymères indéniques tels que les polymères issus de la polymérisation en proportion majoritaire de monomère indène et en proportion minoritaire de monomères choisis parmi le styrène, le méthylindène, le méthylstyrène et leurs mélanges.

- les polymères indéniques tels que les résines de pentadiène et d'indène, qui sont issues de la polymérisation d'un mélange de monomères de pentadiène et d'indène,
les résines de pentadiène telles que celle issues de la polymérisation majoritairement du monomère 1,3-pentadiène (trans ou cis pipérylène) et de monomères minoritaires choisis parmi l'isoprène, le butène, le 2-méthyl-2-butène, le pentène, le 1,4-pentadiène et leurs mélanges,
- les résines des dimères du cyclopentanediène telles que celles issues de la polymérisation de premiers monomères choisis parmi l'indène et le styrène, et de deuxièmes monomères choisis parmi les dimères du cyclopentanediène tels que le dicyclopentadiène, le méthyldicyclopentadiène, les autres dimères du pentanediène, et leurs mélanges ;
- les résines terpéniques issues de la polymérisation d'au moins un monomère choisi parmi l'$\alpha$-pinène, le $\beta$-pinène, le limonène, et leurs mélanges.

5. Produit selon la revendication précédente, **caractérisé en ce que** le polymère hydrocarboné indénique est un copolymère bloc obtenu à partir d'indène, et de styrène ou d'un dérivé du styrène.

6. Produit selon la revendication précédente, **caractérisé en ce que** le polymère hydrocarboné indénique est obtenu par polymérisation en proportion majoritaire de monomère indène et en proportion minoritaire de monomères comme le styrène, le méthylindène, le méthylstyrène et leurs mélanges,

7. Produit selon l'une des revendications précédentes, **caractérisé en ce que** la résine ou le polymère hydrocarboné indénique est choisi parmi les copolymères indène/méthylstyrène/styrène hydrogéné.

8. Produit selon l'une quelconque des revendications précédentes, **caractérisé en ce que** la résine est présente en une teneur allant de 0,1 à 20% en poids par rapport au poids total de la composition, de préférence de 0,5 à 15% en poids et mieux de 1 à 10% en poids.

9. Produit selon l'une des revendications précédentes, **caractérisé en ce que** la première composition comprend une phase grasse liquide.

10. Produit selon la revendication précédente, **caractérisé en ce que** la phase grasse liquide de la première composition comprend au moins une huile volatile hydrocarbonée.

11. Produit selon l'une des revendications 9 ou 10, **caractérisé en ce que** la phase grasse liquide de la première composition comprend un alcool gras.

12. Produit selon l'une des revendications 9 à 11, **caractérisé en ce qu'**elle comprend en outre un copolymère séquencé hydrocarboné, soluble dans ladite phase grasse liquide.

13. Produit selon la revendication précédente, **caractérisé en ce que** le copolymère séquencé hydrocarboné est choisi parmi les copolymères séquencés comprenant au moins un bloc styrène et au moins un bloc comprenant des motifs choisis parmi le butadiène, l'éthylène, le propylène, le butylène, l'isoprène ou un de leurs mélanges.

14. Produit selon la revendication précédente, **caractérisé en ce que** le ratio massique entre la résine et le copolymère séquencé hydrocarboné est compris entre 80/20 et 40/60, notamment entre 75/25 et 50/50.

15. Produit selon l'une des revendications précédentes, **caractérisé en ce que** la première composition et/ou la deuxième composition contient un agent de coloration choisi parmi les colorants liposolubles, les colorants hydrosolubles, les pigments, les nacres et leurs mélanges.

16. Produit cosmétique selon l'une quelconque des revendications précédentes, **caractérisé par le fait que** les première et seconde compositions se présente sous forme de pâte.

17. Produit selon l'une des revendications précédentes, **caractérisé en ce que** la seconde composition est transparente.

18. Produit selon l'une des revendications précédentes, **caractérisé en ce que** la seconde composition contient moins de 5% de pigments, de préférence moins de 2%, de préférence encore moins de 1 %.

19. Produit selon l'une des revendications précédentes, **caractérisé en ce que** le produit présente une brillance supérieure ou égale à 5, en particulier supérieure ou égale à 10, notamment supérieure ou égale à 15, plus particulièrement supérieure ou égale à 20, notamment supérieure ou égale à 25, voire de l'ordre de 30.

**20.** Produit selon l'une des revendications précédentes, **caractérisé en ce que** le corps gras non volatile est une huile visqueuse de viscosité à 25°C comprise entre 100 et 5 000 000 cSt, notamment entre 500 et 1 000 000 cSt, par exemple entre 1 000 et 700 000 cSt, par exemple encore entre 10 000 cSt et 500 000 cSt, par exemple égale à 350 cSt, 60 000 cSt ou 300 000 cSt.

**21.** Produit selon l'une des revendications précédentes, **caractérisé en ce que** le corps gras non volatile est une huile visqueuse présentant une masse moléculaire comprise entre 600 et 2 500 000 g/mol, par exemple entre 700 et 2 000 000 g/mol, par exemple encore entre 1 000 et 500 000 g/mol, par exemple encore entre 10 000 et 300 000 g/mol.

**22.** Produit selon l'une des revendications précédentes, **caractérisé en ce que** la proportion du corps gras non volatile dans la deuxième composition est comprise entre 2 et 100% en poids du poids de la deuxième composition, par exemple de 5 à 80%, voir de 10 à 70%, voir même de 20 à 40% en poids du poids de la composition.

**23.** Produit selon l'une des revendications précédentes, **caractérisé en ce que** le corps gras non volatile est choisi parmi les huiles hydrocarbonées, par exemple le di-isostéaryl malate, les polybutènes ou leurs mélanges.

**24.** Produit selon l'une des revendications précédentes, **caractérisé en ce que** le corps gras non volatile est siliconé.

**25.** Produit selon la revendication précédente, **caractérisé en ce que** le corps gras non volatile est choisie parmi les huiles polyalkylsiloxanes, les polyarylsiloxanes, les polyalkylarylsiloxanes et leurs mélanges.

**26.** Produit selon la revendication précédente, **caractérisé en ce que** le corps gras non volatile est un polymère siliconé de masse moléculaire en poids supérieure ou égale à 200 000 g/mol, de préférence comprise entre 200 000 et 2 500 000, de préférence entre 200 000 et 2 000 000 g/mol.

**27.** Produit selon la revendication précédente, **caractérisé en ce que** le corps gras non volatile est un polymère siliconé de viscosité comprise entre 10 000 et 5 000 000 cSt, de préférence entre 100 000 et 1 000 000 cSt, de préférence encore entre 300 000 et 700 000 cSt.

**28.** Produit selon l'une des revendications précédentes, **caractérisé en ce qu'**il se présente sous la forme d'un fond de teint, d'un fard à joues ou à paupières, d'un rouge à lèvres, d'un produit ayant des propriétés de soin, d'un mascara, d'un eye-liner, d'un vernis à ongles, d'un produit anti-cernes, ou d'un produit de maquillage du corps.

**29.** Procédé de maquillage de la peau et/ou des lèvres et/ou des phanères consistant à appliquer sur la peau, les lèvres et/ou les phanères

- une première couche d'une première composition du produit selon l'une des revendications 1 à 28, et
- une seconde couche d'une seconde composition comprenant une huile visqueuse de viscosité à 20°C supérieure ou égale à 100 cSt, du produit selon l'une des revendications 1 à 28.

**30.** Kit de maquillage comprenant un produit selon l'une des revendications 1 à 28, chacune des première et seconde compositions étant conditionnées de manière séparée dans des premier et second compartiments.

**31.** Utilisation d'un produit cosmétique comprenant une première et une seconde compositions, la première composition comprenant au moins une résine choisie parmi la colophane et ses dérivés, les résines hydrocarbonées et leurs mélanges, ladite résine présentant une masse moléculaire moyenne en nombre inférieure ou égale à 10 000 g/mol, et la seconde composition comprenant un corps gras non volatil, pour conférer à la peau et/ou les lèvres et/ou les phanères un maquillage confortable, brillant, et de bonne tenue.

Office européen des brevets

# RAPPORT DE RECHERCHE EUROPEENNE

Numéro de la demande
EP 06 29 1033

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 1 469 042 A (MERCK PATENT GMBH [DE]) 20 octobre 2004 (2004-10-20) * exemple 8 * | 1-28 | INV. A61K8/04 A61K8/06 A61K8/72 A61K8/81 |
| X | US 5 800 816 A (BRIEVA ET AL) 1 septembre 1998 (1998-09-01) * alinéa [0006]; exemple 1 * | 1-28 | |
| X | EP 1 044 674 A (SHISEIDO COMPANY LIMITED) 18 octobre 2000 (2000-10-18) * tableaux * * alinéas [0002], [0006], [0007] * | 1-31 | |
| X | US 4 699 780 A (JENNINGS ET AL) 13 octobre 1987 (1987-10-13) * colonne 4, ligne 10 - ligne 17; revendication 1; exemples * * page 1, ligne 10 - ligne 40 * | 1-31 | |
| X | FR 2 795 309 A (NIPPON FINE CHEMICAL COMPANY LIMITED) 29 décembre 2000 (2000-12-29) * exemples 1,2,9,13,19,24,25,27 * * page 2, ligne 1 - ligne 6 * * page 5, ligne 11 - ligne 12 * | 1-31 | DOMAINES TECHNIQUES RECHERCHES (IPC) |
| | | | A61K |
| X | EP 0 815 826 A (L'OREAL) 7 janvier 1998 (1998-01-07) * exemple 2 * | 1-28 | |
| X | DE 197 07 309 A1 (LANCASTER GROUP GMBH, 67059 LUDWIGSHAFEN, DE; LANCASTER GROUP GMBH) 13 août 1998 (1998-08-13) * colonne 3, ligne 19 - ligne 24; revendications * | 1-28 | |
| | -/-- | | |

1

Le présent rapport a été établi pour toutes les revendications

| Lieu de la recherche | Date d'achèvement de la recherche | Examinateur |
|---|---|---|
| Munich | 9 octobre 2006 | KRATTINGER, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

Office européen des brevets

# RAPPORT DE RECHERCHE EUROPEENNE

Numéro de la demande
EP 06 29 1033

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | FR 933 963 A (ALBERTE-JEANNE-JULIETTE-LAURE LEMOINE) 7 mai 1948 (1948-05-07) * phrases 1-7,15,16 * ----- | 1-31 | |
| Y | EP 1 518 534 A (L'OREAL) 30 mars 2005 (2005-03-30) * alinéas [0001], [0002], [0014], [0212] - [0217]; exemples 14,15,19,20,27 * ----- | 1-31 | |
| | | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 9 octobre 2006 | KRATTINGER, B |

1

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........................................................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 06 29 1033

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-10-2006

| Document brevet cité au rapport de recherche | Date de publication | Membre(s) de la famille de brevet(s) | Date de publication |
|---|---|---|---|
| EP 1469042 A | 20-10-2004 | CN 1550525 A | 01-12-2004 |
| | | JP 2004292816 A | 21-10-2004 |
| | | US 2004191198 A1 | 30-09-2004 |
| US 5800816 A | 01-09-1998 | AT 223696 T | 15-09-2002 |
| | | AU 709441 B2 | 26-08-1999 |
| | | AU 3439195 A | 09-05-1996 |
| | | BR 9504533 A | 27-05-1997 |
| | | CA 2161285 A1 | 26-04-1996 |
| | | CN 1127631 A | 31-07-1996 |
| | | DE 69528131 D1 | 17-10-2002 |
| | | DE 69528131 T2 | 13-03-2003 |
| | | EP 0709083 A2 | 01-05-1996 |
| | | ES 2182870 T3 | 16-03-2003 |
| | | FI 955048 A | 26-04-1996 |
| | | FR 2726467 A1 | 10-05-1996 |
| | | GB 2294392 A | 01-05-1996 |
| | | HK 1013252 A1 | 12-05-2000 |
| | | IL 115693 A | 13-08-2000 |
| | | JP 8239316 A | 17-09-1996 |
| | | NO 954201 A | 26-04-1996 |
| | | NZ 280302 A | 20-12-1996 |
| | | SG 42788 A1 | 17-10-1997 |
| | | TW 482677 B | 11-04-2002 |
| | | ZA 9509024 A | 06-08-1996 |
| EP 1044674 A | 18-10-2000 | CN 1273085 A | 15-11-2000 |
| | | TW 518231 B | 21-01-2003 |
| | | US 6325996 B1 | 04-12-2001 |
| US 4699780 A | 13-10-1987 | AU 577018 B2 | 08-09-1988 |
| | | AU 5768386 A | 04-12-1986 |
| | | CA 1254147 A1 | 16-05-1989 |
| | | DE 3676353 D1 | 07-02-1991 |
| | | EP 0205961 A2 | 30-12-1986 |
| | | JP 2014666 C | 02-02-1996 |
| | | JP 7045379 B | 17-05-1995 |
| | | JP 61277606 A | 08-12-1986 |
| FR 2795309 A | 29-12-2000 | AUCUN | |
| EP 0815826 A | 07-01-1998 | BR 9702553 A | 29-09-1998 |
| | | CA 2209435 A1 | 02-01-1998 |
| | | FR 2750599 A1 | 09-01-1998 |
| | | JP 3001831 B2 | 24-01-2000 |
| | | JP 10072317 A | 17-03-1998 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO.

EP 06 29 1033

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-10-2006

| Document brevet cité au rapport de recherche | Date de publication | Membre(s) de la famille de brevet(s) | Date de publication |
|---|---|---|---|
| DE 19707309 A1 | 13-08-1998 | AT 210964 T | 15-01-2002 |
| | | AU 6717998 A | 26-08-1998 |
| | | CA 2278685 A1 | 13-08-1998 |
| | | CN 1246787 A | 08-03-2000 |
| | | CZ 9902809 A3 | 17-11-1999 |
| | | WO 9834588 A1 | 13-08-1998 |
| | | EP 0975320 A1 | 02-02-2000 |
| | | ES 2170485 T3 | 01-08-2002 |
| | | HU 0000680 A2 | 28-04-2001 |
| | | JP 2001511161 T | 07-08-2001 |
| | | PL 334680 A1 | 13-03-2000 |
| FR 933963 A | 07-05-1948 | AUCUN | |
| EP 1518534 A | 30-03-2005 | CN 1615811 A | 18-05-2005 |
| | | FR 2860142 A1 | 01-04-2005 |
| | | JP 2005126417 A | 19-05-2005 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 847752 A
- EP 1396259 A
- EP 1068854 A
- EP 1086945 A
- WO 0247031 A
- US 5874069 A
- US 5919441 A
- US 6051216 A
- US 5981680 A
- US 4152416 A
- EP 0667146 A

### Littérature non-brevet citée dans la description

- Handbook of Pressure Sensitive Adhesive. 1989, 609-619
- Polymer Handbook. John Wiley, 1989
- **P. TERECH.** Specialist Surfactants. 1997, 209-263